# EUROPÄISCHE PATENTSCHRIFT

(11) **EP 3 868 317 B1**
(45) Veröffentlichungstag und Bekanntmachung des Hinweises auf die Patenterteilung: **22.07.2026**
(21) Anmeldenummer: 21154630.4
(22) Anmeldetag: 01.02.2021
(51) Int. Cl.: A61B 17/34, A61B 1/00, A61B 1/012, A61B 90/98, A61B 1/07, A61B 1/12

(54) **MEDIZINISCHES SYSTEM UND TROKAR**
MEDICAL SYSTEM AND TROCAR
SYSTÈME MÉDICAL TROCART

(30) Priorität: 21.02.2020 DE 102020104574
(43) Veröffentlichungstag der Anmeldung: 25.08.2021
(73) Patentinhaber: OLYMPUS WINTER & IBE GMBH, 22045 Hamburg (DE)
(72) Erfinder: Wieters, Martin, 22885 Barsbüttel (DE)
(74) Vertreter: Noack, Andreas

(56) Entgegenhaltungen:
- WO-A1-2018/232360
- DE-A1- 102011 088 336
- DE-A1- 102012 213 205
- DE-A1- 102016 124 730
- DE-U1- 202007 015 093
- ES-T3- 2 339 768
- US-A1- 2017 086 906

## Beschreibung

Die Erfindung betrifft ein medizinisches System mit einem medizinischen Instrument und einem Trokar, wobei der Trokar vorgesehen ist, einen künstlichen Zugang zu einer Körperhöhle eines Patienten bereitzustellen, und das medizinische Instrument vorgesehen ist, durch den Trokar in die Körperhöhle des Patienten eingeführt zu werden und in der Körperhöhle des Patienten eine medizinische Funktion auszuführen.

Weiterhin offenbart wird eine Medien- und/oder Energiequelle sowie einen Trokar eines medizinischen Systems.

Medizinische Systeme der eingangs genannten Art werden in der modernen Medizin verwendet, um Eingriffe in Körperhöhlen eines Patienten durchzuführen, ohne diese großräumig zu eröffnen. Entsprechende Eingriffe werden daher auch als minimalinvasive Eingriffe bezeichnet.

Bei minimalinvasiven Eingriffen erfolgt die Kontrolle des Eingriffs in der Regel mittels eines Endoskops, welches durch einen Trokar in die Körperhöhle eingeführt wird, und welches ein Videobild der Körperhöhle aufnimmt. Das Videobild wird auf einem Monitor angezeigt, so dass ein behandelnder Arzt sich im OP-Feld orientieren kann.

Um mit einem Endoskop ein Bild einer Körperhöhle aufnehmen zu können ist es erforderlich, das Blickfeld des Endoskops zu beleuchten. Dazu weisen Endoskope üblicherweise ein Beleuchtungssystem aus Lichtleitfasern auf, welche von einem proximalen Ende zu einem distalen Ende des Endoskops verlegt sind. Am proximalen Ende des Endoskops kann Beleuchtungslicht eingekoppelt werden, so dass es am distalen Ende des Endoskops abgestrahlt wird und das Blickfeld des Endoskops ausleuchtet.

Das Beleuchtungslicht wird in der Regel in einer externen Lichtquelle erzeugt, welche über ein Lichtleitkabel mit dem Endoskop verbunden wird. Als Leuchtmittel der Lichtquelle werden vermehrt LEDs oder Laserdioden verwendet. Laserdioden kommen hauptsächlich als Quellen für Anregungslicht bei Fluoreszenzuntersuchungen zum Einsatz, werden vereinzelt aber auch zur Erzeugung von weißem Beleuchtungslicht genutzt.

Bei dem Einsatz von Laserdioden ist jedoch zu beachten, dass die von diesen ausgehende Laserstrahlung eine so hohe Intensität aufweisen kann, dass sie bei direktem Auftreffen auf ein ungeschütztes Auge zu Verletzungen führen kann. Während dies kein Problem ist, solange das Endoskop durch den Trokar in die Körperhöhle eingeführt ist, kann es zu Unfällen kommen, wenn das Endoskop zu Beginn oder am Ende eines Eingriffs noch nicht, oder nicht mehr, in die Körperhöhle eingeführt ist, sondern von einem Arzt frei in der Hand gehalten wird. Ebenso kann es zu Unfällen kommen, wenn das Endoskop während eines Eingriffs kurz aus der Körperhöhle entnommen wird, um beispielsweise die Optik zu reinigen. Die Patentanmeldung US 2017/086906 A1 sieht vor, dass der Trokar eines medizinischen Systems mit einem Informationsdetektor ausgestattet ist und das Durchführen eines medizinischen Instruments anhand einer auf dem Endoskopschaft aufgebrachten Identifizierungsinformation feststellt wird, woraufhin eine Energiequelle freigegeben wird. Einige Endoskope weisen eine Spülvorrichtung für die Optik auf. In diese Spülvorrichtung kann von einer externen Quelle ein Spülmedium, beispielsweise Wasser, eingeführt werden, welches dass in Richtung der Optik abgegeben wird, um diese zu reinigen. Wird jedoch die externe Medienquelle aktiviert, wenn das Endoskop gerade nicht in den Trokar eingeführt ist, so kann es zu einer unkontrollierten Abgabe des Mediums im OP-Saal kommen. Dies führt einerseits zu unnötigen Verschmutzungen und kann andererseits ein weiteres Umfallrisiko darstellen, z.B. durch Ausrutschen.

Die Patentanmeldung DE 10 2016 124730 A1 offenbart einen Trokar mit einem "signal interface adapter", welcher am Trokar angeordnet ist, und einem "instrument connector", welcher über eine Feder so am Handgriff eines elektrochirurgischen Instruments angeordnet ist, dass er beim Einführen des Instruments in den Trokar mit dem "signal interface adapter" in Kontakt tritt.

Es besteht daher die Aufgabe der Erfindung darin, ein medizinisches System bereitzustellen, welches hinsichtlich der beschriebenen Problematiken verbessert ist.

Diese Aufgabe wird gemäß eines Aspekts der Erfindung gelöst durch ein medizinisches System mit einem medizinischen Instrument und einem Trokar, wobei der Trokar vorgesehen ist, einen künstlichen Zugang zu einer Körperhöhle eines Patienten bereitzustellen, und das medizinische Instrument vorgesehen ist, durch den Trokar in die Körperhöhle des Patienten eingeführt zu werden und in der Körperhöhle des Patienten eine medizinische Funktion auszuführen, welches dadurch weitergebildet ist, dass das medizinische System eine Steuerung umfasst, die eingerichtet ist, festzustellen, ob das medizinische Instrument in den Trokar eingeführt ist, und das Ausführen einer medizinischen Funktion durch das medizinische Instrument nur freizugeben, wenn das medizinische Instrument in den Trokar eingeführt ist.

Durch die erfindungsgemäße Ausführung des medizinischen Systems ist sichergestellt, dass die medizinische Funktion nur dass ausgeführt wird, wenn das medizinische Instrument in den Trokar eingeführt ist. Eine ungewollte negative Auswirkung der medizinischen Funktion außerhalb der zu behandelnden bzw. zu untersuchenden Körperhöhle kann somit vermieden werden.

Gemäß einer vorteilhaften Weiterbildung der Erfindung kann das medizinische System weiterhin eine Medien- und/oder Energiequelle umfassen, welche zur Bereitstellung von Medien und/oder Energie für die Ausführung der medizinischen Funktion vorgesehen ist, und die Steuerung kann eingerichtet sein, die Bereitstellung von Medien und/oder Energie nur freizugeben, wenn das medizinische Instrument in den Trokar eingeführt ist. Hierdurch kann insbesondere die ungewollte Abgabe von Energie oder Medien außerhalb der zu behandelnden bzw. zu untersuchenden Körperhöhle verhindert werden.

Das medizinische Instrument des medizinischen Systems ist gemäß der Erfindung ein Endoskop.

Die Medienquelle kann zur Bereitstellung von Spülflüssigkeit vorgesehen sein. Dabei wird die Abgabe von Spülflüssigkeit außerhalb der zu untersuchenden bzw. zu behandelnden Körperhöhle sowie damit verbundene Probleme und Risiken vermieden.

Die Energiequelle kann eine Lichtquelle umfassen. Vorzugsweise kann die Energiequelle eine Laserlichtquelle umfassen. Licht, insbesondere Laserlicht, kann bei ungewollter Abgabe außerhalb einer zu untersuchenden bzw. zu behandelnden Körperhöhle leicht zu Unfällen und Verletzungen führen. Durch die Erfindung kann hier die Betriebssicherheit eines medizinischen Systems deutlich gesteigert werden.

In einem medizinischen System nach der Erfindung weist das Endoskop einen Schaft mit leitfähiger Oberfläche auf, und der Trokar weist wenigstens einen elektrischen Kontakt auf, welcher den Schaft des Endoskops berührt, wenn dieses in den Trokar eingeführt ist.

Dabei kann der Trokar besonders bevorzugt zwei elektrische Kontakte aufweisen, welche durch den Schaft des Endoskops miteinander verbunden werden, wenn das Endoskop in den Trokar eingeführt ist. Auf diese Weise kann das Einführen des Endoskops in den Trokar besonders unkompliziert festgestellt werden.

In einer möglichen Ausführung eines medizinisches Systems nach der Erfindung kann der wenigstens eine elektrische Kontakt Bestandteil einer Dichtung des Trokars sein. Dabei kann der wenigstens eine elektrische Kontakt durch ein Dichtelement aus einem elektrisch leitfähigem elastischen Polymer gebildet sein. Bei einer entsprechenden Ausführung ist der mechanische Aufwand besonders gering.

In einer weiteren möglichen Ausführung eines medizinischen Systems, die nicht Bestandteil der Erfindung ist, kann das Endoskop einen Schaft aus einem magnetischen Material aufweisen, und der Trokar kann wenigstens eine Spule umfassen, deren Induktivität verändert ist, wenn das Endoskop in den Trokar eingeführt ist. Auf diese Weise kann das Einführen des Endoskops in den Trokar berührungslos festgestellt werden.

In einer weiteren möglichen Ausführung eines medizinischen Systems, die nicht Bestandteil der Erfindung ist, kann der Trokar einen Kondensator umfassen, dessen Kapazität verändert ist, wenn das Endoskop in den Trokar eingeführt ist. Auch auf diese Weise kann das Einführen des Endoskops in den Trokar berührungslos festgestellt werden.

In einer zusätzlichen möglichen Ausführung eines medizinischen Systems, die nicht Bestandteilder Erfindung ist, kann der Trokar einen Tastschalter umfassen, der betätigt wird, wenn das Endoskop in den Trokar eingeführt ist. Auf diese Weise kann das Einführen des Endoskops in den Trokar ebenfalls besonders einfach festgestellt werden.

Die Steuerung eines medizinischen Systems gemäß der Erfindung kann vorzugsweise eine in dem Trokar integrierte Schaltung umfassen, welche feststellt, ob das medizinische Instrument in den Trokar eingeführt ist. Besonders vorzugsweise kann die Steuerung eingerichtet sein, ein Freigabesignal drahtlos oder drahtgebunden bereitzustellen, wenn das medizinische Instrument in den Trokar eingeführt ist. Durch die Integration der Schaltung in den Trokar kann die Erfindung umgesetzt werden, ohne dass ein separates Gerät benötigt wird.

Gemäß eines weiteren Aspekts der Erfindung wird die Aufgabe gelöst durch einen Trokar eines entsprechenden medizinischen Systems.

Hinsichtlich der hierdurch erreichbaren Vorteile und Wirkungen wird auf die obigen Ausführungen ausdrücklich verwiesen.

Die Erfindung wird nachfolgend anhand einiger beispielhafter Zeichnungen näher erläutert. Dabei sollen die dargestellten Ausführungsbeispiele lediglich zum besseren Verständnis der Erfindung beitragen, ohne diese einzuschränken.

Es zeigen:
Fig. 1: ein medizinisches System,
Fig. 2: einen Trokar,
Fig. 3: einen weiteren Trokar,
Fig. 4: noch einen weiteren Trokar.

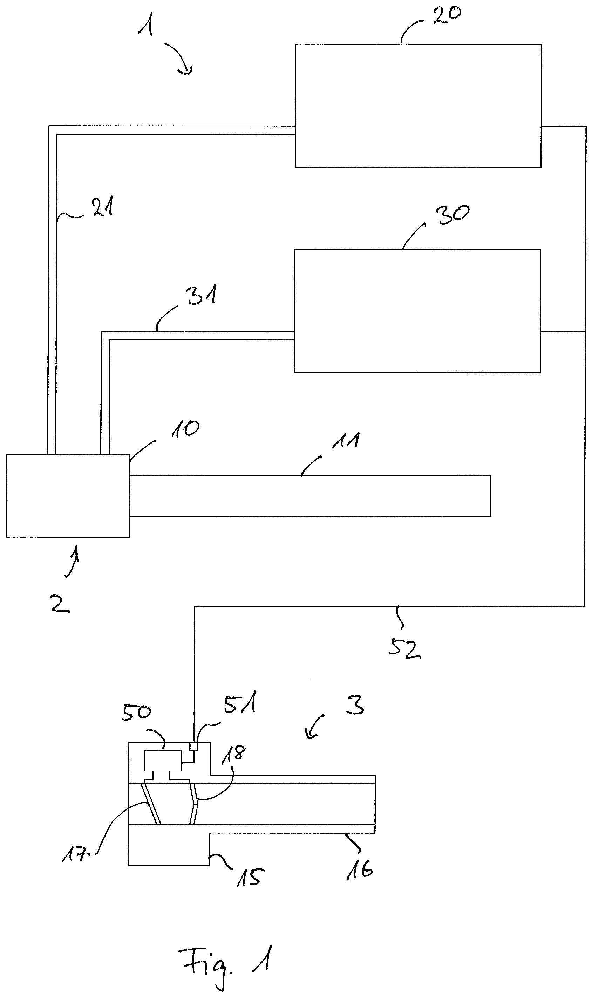

Figur 1 zeigt ein medizinisches System 1. Das medizinische System 1 umfasst ein medizinisches Instrument in Form eines Endoskops 2, sowie einen Trokar 3.

Das Endoskop 2 weist einen Hauptkörper 10 sowie einen langgestreckten Schaft 11 auf. Der Schaft 11 und vorzugsweise auch der Hauptkörper 10 bestehen aus einem biokompatiblen metallischen Werkstoff, beispielsweise medizinischem Stahl.

Der Trokar 3 weist einen Trokarkopf 15 und eine Kanüle 16 auf, die beispielsweise aus einem biokompatiblen Kunststoff wie PEEK bestehen können.

Das medizinische System 1 wird bei minimalinvasiven chirurgischen Prozeduren eingesetzt, um ein Bild von dem Operationssitus bereitzustellen. Dazu wird der Trokar 3 durch eine Inzision in eine zu behandelnde oder zu untersuchende Körperhöhle (nicht dargestellt) eingeführt, wobei der Trokarkopf 15 außerhalb des Patienten verbleibt und dort fixiert werden kann. Anschließend wird der Schaft 11 des Endoskops 2 durch den Trokar 3 in die Körperhöhle eingeführt.

Um einen Eingriff in der Körperhöhle zu ermöglichen wird diese mit einem Gas, beispielsweise CO₂, aufgedehnt. Damit das Gas nicht durch den Trokar 3 entweicht, ist dieser mit Dichtungen 17, 18 ausgestattet. Dabei ist die Dichtung 17 als Verschlussdichtung ausgeführt, welche den Trokar 3 vollständig verschließt, wenn kein Instrument eingeführt ist. Die Dichtung 18 kann eine Lippendichtung sein, welche sich bei eingeführtem Instrument an den Schaft des Instruments anlegt. Die Dichtungen 17, 18 bestehen aus einem biokompatiblen elastischen Polymer wie beispielsweise Silikon.

Das medizinische System 1 umfasst weiterhin eine Energiequelle in Form einer Lichtquelle 20. Die Lichtquelle 20 ist über ein Lichtleitkabel 21 mit dem Endoskop 2 verbunden. Im Betrieb wird Licht von der Lichtquelle 20 über das Lichtleitkabel 21 in das Endoskop geleitet, wo es über nicht dargestellte Lichtleiter zu einem distalen Ende des Schaftes 11 weitergeleitet wird. Am distalen Ende des Schafts 11 wird das Licht abgestrahlt, um ein Blickfeld des Endoskops 2 auszuleuchten.

Die Lichtquelle 20 erzeugt intensives weißes Licht, welches ein ungeschütztes Auge schädigen kann. Dies gilt insbesondere, wenn die Lichtquelle eine Laserlichtquelle beinhaltet, wie beispielsweise in der DE102016124730A1 beschrieben. Laserlichtquellen werden auch zur Erzeugung von Anregungslicht bei Fluoreszenzuntersuchungen verwendet.

Das medizinische System 1 umfasst weiterhin eine Medienquelle in Form einer Pumpe 30 für Spülflüssigkeit. Die Pumpe 30 ist über einen Spülschlauch mit dem Endoskop 2 verbunden. Spülflüssigkeit wird von der Pumpe 30 durch den Spülschlauch in das Endoskop 2 gefördert und dort in Richtung einer nicht dargestellten Optik abgegeben, um diese während des Eingriffs von Verschmutzungen zu säubern.

Spülflüssigkeit aus dem Endoskop 3 kann zu Verschmutzungen in einem OP-Saal führen, darüber hinaus kann durch Spülflüssigkeit eine Unfallgefahr entstehen, z.B. durch Ausrutschen.

Das medizinische System weist eine Steuerung auf, welche die Abgabe von Energie und/oder Spülflüssigkeit durch die Lichtquelle 20 bzw. die Pumpe 30 verhindert, solange das Endoskop 2 nicht in den Trokar 3 eingeführt ist.

Bei den in Figur 1 dargestellten Ausführung sind die Dichtungen 17, 18 aus elektrisch leitfähigem Polymer gefertigt. Hierzu kann das Polymer der Dichtungen 17, 18 mit einem Metallpulver versetzt oder mit einer geeigneten Beschichtung versehen sein. Die Dichtungen 17, 18 sind mit einer Schaltung 50 verbunden.

Wenn das Endoskop 2 in den Trokar 3 eingeführt ist, wird durch den metallischen Schaft 11 eine elektrische Verbindung zwischen den Dichtungen 17, 18 hergestellt. Dies wird durch die Schaltung 50 erkannt. Die Schaltung 50 erzeugt daraufhin ein Freigabesignal, welches an einer Schnittstelle 51 bereitgestellt wird. Bei der Schnittstelle 51 kann es sich beispielsweise um einen Steckkontakt oder eine drahtlose Schnittstelle handeln. Als drahtlose Schnittstelle kommt beispielsweise eine Bluetooth- oder WLAN-Schnittstelle in Betracht. Bei einem Steckkontakt kann das Freigabesignal über eine Signalleitung 52 übermittelt werden.

Die Lichtquelle 20 und die Pumpe 30 sind eingerichtet, das Freigabesignal von der Schaltung 50 zu empfangen, und nur Licht bzw. Spülflüssigkeit bereitzustellen, wenn das Freigabesignal vorliegt. Auf diese Weise wird sicher verhindert, dass die Lichtquelle 20 oder die Pumpe 30 aktiviert werden, wenn das Endoskop 2 nicht durch den Trokar 3 in die zu untersuchende oder zu behandelnde Körperhöhle eingeführt ist. Wird das Endoskop 2 während des Eingriffs aus dem Trokar 3 herausgezogen, beispielsweise um es gründlicher zu reinigen, so wird dies ebenso durch die Schaltung 50 erkannt, und die Lichtquelle 20 und/oder die Pumpe 30 können sicher deaktiviert werden, bevor ein Unfallrisiko entsteht.

Wenn die Lichtquelle 20 eingerichtet ist, sowohl Weißlicht als auch Laserlicht bereitzustellen, kann die Bereitstellung von Weißlicht unabhängig von dem Freigabesignal erfolgen, während Laserlicht nur bereitgestellt wird, wenn das Freigabesignal vorliegt.

In dem dargestellten medizinischen System 1 weist der Trokar 3 zwei elektrische Kontakte auf. Prinzipiell kann das Einführen des Endoskops 2 in den Trokar 3 auch mit einem einzelnen Kontakt ermittelt werden. Dazu kann ermittelt werden, ob über die Signalleitung 51 und das Lichtleitkabel 21 bzw. den Spülschlauch 31 und den Schaft 11 des Endoskops 2 ein Stromkreis geschlossen wird. Hierzu können in dem Lichtleitkabel 21 und/oder der Spülleitung 31 ebenfalls elektrische Leiter vorgesehen sein.

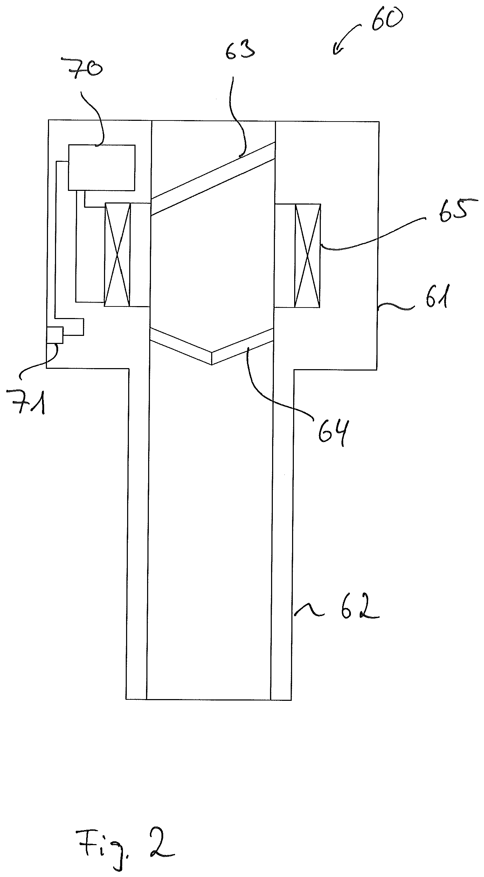

In Figur 2 ist ein Trokar 60 dargestellt, welcher alternativ zu dem Trokar 3 in dem medizinischen System 1 verwendet werden kann.

Der Trokar 60 umfasst wiederum einen Trokarkopf 61 sowie eine Kanüle 62, welche durch Dichtungen 63, 64 verschlossen ist. Im Gegensatz zu den Dichtungen 17, 18 sind die Dichtungen 63, 64 nicht bzw. nicht zwangsläufig elektrisch leitend ausgeführt.

In dem Trokarkopf 61 ist eine Spule 65 so angeordnet, dass das in Figur 2 nicht dargestellte Endoskop 2 bei dem Einführen in den Trokar 60 durch die Spule 65 geführt werden muss. Dabei verändert sich durch das magnetische, bevorzugt ferromagnetische Verhalten des Schafts 11 des Endoskops 2 die Induktivität der Spule 65.

Der Trokar 60 umfasst ferner eine Schaltung 70, welche die Induktivität der Spule 65 misst. Wenn die Induktivität durch den Schaft 11 verändert wird, erzeugt die Schaltung 70 ein Freigabesignal und gibt dies über eine Schnittstelle 71 aus. Die Schnittstelle 71 kann ähnlich wir die Schnittstelle 51 beispielsweise ein Steckkontakt oder eine drahtlose Schnittstelle sein.

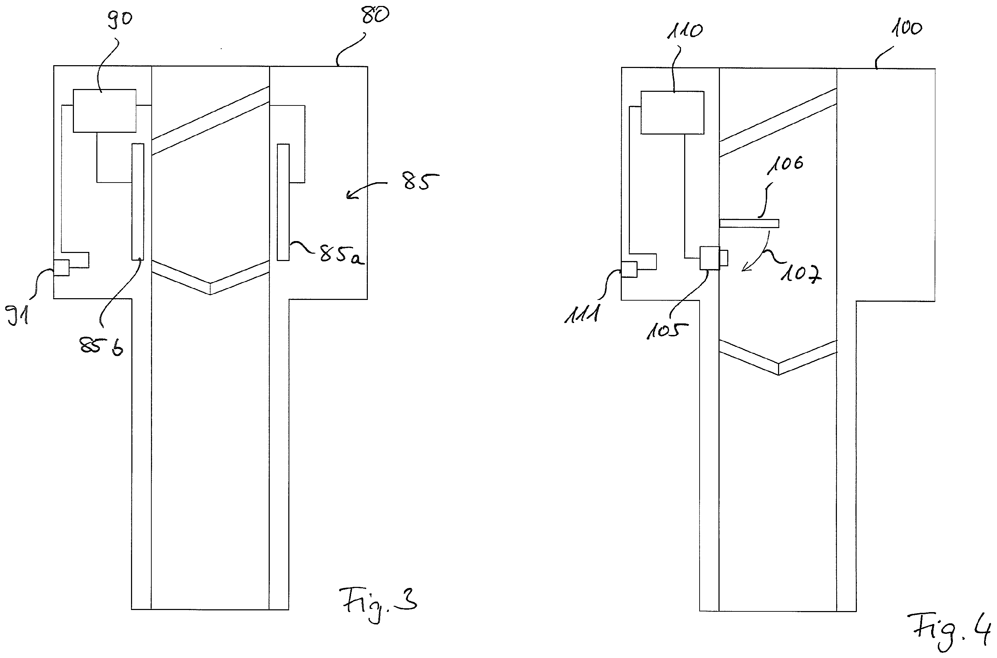

In Figur 3 ist ein weiterer Trokar 80 dargestellt, der ähnlich wie der Trokar 60 ausgeführt ist. Der Trokar 80 umfasst einen Kondensator 85 mit zwei Kondensatorplatten 85a, 85b. Der Kondensator 85 ist so angeordnet, dass ein in Figur 3 nicht dargestelltes Endoskop beim Einführen in den Trokar 80 in die Nähe der Kondensatorplatten 85a, 85b gerät, vorzugsweise zwischen die Kondensatorplatten 85a, 85b. Hierdurch wird die Kapazität des Kondensators 85 verändert.

Der Trokar 80 umfasst eine Schaltung 90, welche die Kapazität des Kondensators 85 misst. Wenn die Kapazität verändert wird, erzeugt die Schaltung 90 ein Freigabesignal und gibt dies über eine Schnittstelle 91 aus. Die Schnittstelle 91 kann ähnlich wir die Schnittstelle 51 beispielsweise ein Steckkontakt oder eine drahtlose Schnittstelle sein.

In Figur 4 ist noch ein weiterer Trokar 100 dargestellt, der ähnlich wie die zuvor beschriebenen Trokare 3, 60, 80 ausgeführt ist. Der Trokar 100 umfasst einen Tastschalter 105, welcher beim Einführen eines in Figur 4 nicht dargestellten Endoskops in den Trokar 100 betätigt wird. Dazu kann eine Klappe 106 in dem Trokar 100 angeordnet sein, die durch das Endoskop in Richtung des Pfeils 107 umgeklappt wird und den Tastschalter 105 betätigt. Die Klappe 106 kann durch eine nicht dargestellte Feder gegen die Richtung des Pfeils 107 vorgespannt sein, so dass sie sich nach Entnehmen des Endoskops aus dem Trokar 100 in die in Figur 4 dargestellte Lage zurückbewegt.

Der Trokar 100 umfasst eine Schaltung 110, welche die Schaltposition des Tastschalters 105 auswertet. Wenn der Tastschalter 105 betätigt wird, erzeugt die Schaltung 110 ein Freigabesignal und gibt dies über eine Schnittstelle 111 aus. Die Schnittstelle 111 kann ähnlich wir die Schnittstellen 51, 71 beispielsweise ein Steckkontakt oder eine drahtlose Schnittstelle sein.

Die Trokare 3, 60, 80, 100 können wiederverwendbar ausgeführt sein. Dazu müssen die Schaltungen 50, 70, 90, 110 ausreichend von Beschädigungen bei einem Wiederaufbereitungsverfahren geschützt werden, was aufwendig sein kann. Alternativ können die Trokare 3, 60, 80, 100 zur Einmalverwendung vorgesehen sein. Die Schaltungen 50, 70 können in diesem Fall relativ kostengünstig als gedruckte Schaltungen ausgeführt sein, und müssen gegen Einwirkungen eines Aufbereitungsvorgangs nicht geschützt werden.

Als Energieversorgung für die Schaltungen 50, 70, 90, 110 kann eine einfache Batterie vorgesehen sein, beispielsweise eine Knopfzelle. Alternativ ist auch eine nicht dargestellte externe Energieversorgung möglich.

## Patentansprüche

1. Medizinisches System mit einem medizinischen Instrument (2) und einem Trokar (3, 60), wobei
- das medizinische Instrument (2) ein Endoskop ist,
- der Trokar (3, 60, 80, 100) vorgesehen ist, einen künstlichen Zugang zu einer Körperhöhle eines Patienten bereitzustellen, und
- das medizinische Instrument (2) vorgesehen ist, durch den Trokar (3, 60, 80, 100) in die Körperhöhle des Patienten eingeführt zu werden und in der Körperhöhle des Patienten eine medizinische Funktion auszuführen,
- das medizinische System eine Steuerung umfasst, die eingerichtet ist, festzustellen, ob das medizinische Instrument (2) in den Trokar (3, 60, 80, 100) eingeführt ist, und
- das Ausführen einer medizinischen Funktion durch das medizinische Instrument (2) nur freizugeben, wenn das medizinische Instrument (2) in den Trokar (3, 60, 80, 100) eingeführt ist,
**dadurch gekennzeichnet, dass**
das Endoskop (2) einen Schaft (11) mit leitfähiger Oberfläche aufweist, und dass der Trokar (3) wenigstens einen elektrischen Kontakt (17,18) aufweist, welcher den Schaft (11) des Endoskops (2) berührt, wenn dieses in den Trokar (3) eingeführt ist.

2. Medizinisches System nach Anspruch 1, **dadurch gekennzeichnet, dass** das System weiterhin eine Medien- und/oder Energiequelle (20, 30) umfasst, welche zur Bereitstellung von Medien und/oder Energie für die Ausführung der medizinischen Funktion vorgesehen ist, und dass die Steuerung eingerichtet ist, die Bereitstellung von Medien und/oder Energie nur freizugeben, wenn das medizinische Instrument (2) in den Trokar (3, 60, 80, 100) eingeführt ist.

3. Medizinisches System nach Anspruch 2, **dadurch gekennzeichnet, dass** die Medienquelle (30) zur Bereitstellung von Spülflüssigkeit vorgesehen ist.

4. Medizinisches System nach Anspruch 2, **dadurch gekennzeichnet, dass** die Energiequelle eine Lichtquelle (20) umfasst.

5. Medizinisches System nach Anspruch 4, **dadurch gekennzeichnet, dass** die Energiequelle eine Laserlichtquelle umfasst.

6. Medizinisches System nach einem der Ansprüche 1 bis 5, **dadurch gekennzeichnet, dass** der Trokar (3) zwei elektrische Kontakte aufweist, welche durch den Schaft (11) des Endoskops (2) miteinander verbunden werden, wenn das Endoskop (2) in den Trokar (3) eingeführt ist.

7. Medizinisches System nach einem der Ansprüche 1 bis 6, **dadurch gekennzeichnet, dass** der wenigstens eine elektrische Kontakt Bestandteil einer Dichtung (17,18) des Trokars (3) ist.

8. Medizinisches System nach Anspruch 7, **dadurch gekennzeichnet, dass** der wenigstens eine elektrische Kontakt durch ein Dichtelement (17,18) aus einem elektrisch leitfähigem elastischen Polymer gebildet ist.

9. Medizinisches System nach einem der vorangehenden Ansprüche, **dadurch gekennzeichnet, dass** die Steuerung eine in dem Trokar (3, 60, 80, 100) integrierte Schaltung (50, 70, 90, 110) umfasst, welche feststellt, ob das medizinische Instrument (2) in den Trokar (3, 60, 80, 100) eingeführt ist.

10. Medizinisches System nach Anspruch 9, **dadurch gekennzeichnet, dass** die Steuerung eingerichtet ist, ein Freigabesignal drahtlos oder drahtgebunden bereitzustellen, wenn das medizinische Instrument (2) in den Trokar (3, 60, 80, 100) eingeführt ist.

11. Trokar eines medizinischen Systems nach einem der Ansprüche 1 bis 10.

## Claims

1. Medical system comprising a medical instrument (2) and a trocar (3, 60), wherein
- the medical instrument (2) is an endoscope,
- the trocar (3, 60, 80, 100) is designed to provide artificial access to a patient's body, and
- the medical instrument (2) is intended to be inserted through the trocar (3, 60, 80, 100) into the patient's body cavity and to perform a medical function within the patient's body cavity,
- the medical system comprises a control unit configured to determine whether the medical instrument (2) is inserted into the trocar (3, 60, 80, 100), and
- to authorise the performance of a medical function by the medical instrument (2) only when the medical instrument (2) is inserted into the trocar (3, 60, 80, 100),
**characterised in that**
the endoscope (2) comprises a shaft (11) with a conductive surface, and that the trocar (3) comprises at least one electrical contact (17, 18) which contacts the shaft (11) of the endoscope (2) when the latter is inserted into the trocar (3).

2. Medical system according to claim 1, **characterised in that** the system further comprises a media and/or energy source (20, 30) intended to supply media and/or energy for the performance of the medical function, and **in that** the control is configured to authorise the supply of media and/or energy only when the medical instrument (2) is inserted into the trocar (3, 60, 80, 100).

3. Medical system according to claim 2, **characterised in that** the media source (30) is intended to supply irrigation fluid.

4. Medical system according to claim 2, **characterised in that** the energy source comprises a light source (20).

5. Medical system according to claim 4, **characterised in that** the energy source comprises a laser light source.

6. Medical system according to any one of claims 1 to 5, **characterised in that** the trocar (3) has two electrical contacts which are connected to one another via the shaft (11) of the endoscope (2) when the endoscope (2) is inserted into the trocar (3).

7. Medical system according to any one of claims 1 to 6, **characterised in that** the at least one electrical contact forms part of a seal (17, 18) of the trocar (3).

8. Medical system according to claim 7, **characterised in that** the at least one electrical contact is formed by a sealing element (17, 18) made of an electrically conductive elastic polymer.

9. Medical system according to any one of the preceding claims, **characterised in that** the control unit comprises a circuit (50, 70, 90, 110) integrated into the trocar (3, 60, 80, 100), which determines whether the medical instrument (2) is inserted into the trocar (3, 60, 80, 100).

10. Medical system according to claim 9, **characterised in that** the control unit is configured to provide an enable signal wirelessly or via a wired connection when the medical instrument (2) is inserted into the trocar (3, 60, 80, 100).

11. Trocar of a medical system according to any one of claims 1 to 10.

## Revendications

1. Système médical comprenant un instrument médical (2) et un trocart (3, 60), dans lequel
- l'instrument médical (2) est un endoscope,
- le trocart (3, 60, 80, 100) est prévu pour créer un accès artificiel à une cavité corporelle d'un patient, et
- l'instrument médical (2) est prévu pour être introduit dans la cavité corporelle du patient à travers le trocart (3, 60, 80, 100) et pour exercer une fonction médicale dans la cavité corporelle du patient,
- le système médical comprend une commande qui est agencée pour déterminer si l'instrument médical (2) est introduit dans le trocart (3, 60, 80, 100), et
- de n'autoriser l'exécution d'une fonction médicale par l'instrument médical (2) que lorsque l'instrument médical (2) est introduit dans le trocart (3, 60, 80, 100),
**caractérisé en ce que**
l'endoscope (2) comporte une tige (11) à surface conductrice, et **en ce que** le trocart (3) comporte au moins un contact électrique (17, 18) qui entre en contact avec la tige (11) de l'endoscope (2) lorsque celui-ci est inséré dans le trocart (3).

2. Système médical selon la revendication 1, **caractérisé en ce que** le système comprend en outre une source de médium et/ou d'énergie (20, 30) destinée à fournir un médium et/ou de l'énergie pour l'exécution de la fonction médicale, et **en ce que** la commande est agencée pour n'autoriser la fourniture de médium et/ou d'énergie que lorsque l'instrument médical (2) est inséré dans le trocart.

3. Système médical selon la revendication 2, **caractérisé en ce que** la source de médium (30) est prévue pour fournir un liquide de rinçage.

4. Système médical selon la revendication 2, **caractérisé en ce que** la source d'énergie comprend une source de lumière (20).

5. Système médical selon la revendication 4, **caractérisé en ce que** la source d'énergie comprend une source de lumière laser.

6. Système médical selon l'une des revendications 1 à 5, **caractérisé en ce que** le trocart (3) comporte deux contacts électriques qui sont reliés l'un à l'autre par la tige (11) de l'endoscope (2) lorsque l'endoscope (2) est introduit dans le trocart (3).

7. Système médical selon l'une des revendications 1 à 6, **caractérisé en ce que** le au moins un contact électrique fait partie intégrante d'un joint d'étanchéité (17, 18) du trocart (3).

8. Système médical selon la revendication 7, **caractérisé en ce que** le au moins un contact électrique est formé par un élément d'étanchéité (17, 18) en polymère élastique électriquement conducteur.

9. Système médical selon l'une des revendications précédentes, **caractérisé en ce que** la commande comprend un circuit (50, 70, 90, 110) intégré dans le trocart (3, 60, 80, 100), qui détecte si l'instrument médical (2) est inséré dans le trocart (3, 60, 80, 100).

10. Système médical selon la revendication 9, **caractérisé en ce que** la commande est agencée pour fournir un signal de validation par voie sans fil ou filaire lorsque l'instrument médical (2) est inséré dans le trocart (3, 60, 80, 100).

11. Trocart d'un système médical selon l'une des revendications 1 à 10.
